# EUROPEAN PATENT APPLICATION

(11) **EP 4 799 615 A1**
(43) Date of publication of application: **02.09.2026**
(21) Application number: 24881675.3
(22) Date of filing: 24.10.2024
(51) Int. Cl.: A61K 31/18, A61K 9/00, A61P 9/00, A61P 25/00, A61K 47/02

(54) **PHARMACEUTICAL COMPOSITION OF SULFONYLUREA DERIVATIVE**

(30) Priority: 24.10.2023 CN 202311382830
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: YU, Shuxiang, Lianyungang, Jiangsu 222047 (CN); SHI, Xiaolei, Lianyungang, Jiangsu 222047 (CN); YANG, Xiaorong, Lianyungang, Jiangsu 222047 (CN); WANG, Xinyue, Shanghai 200245 (CN); ZHANG, Yujie, Shanghai 200245 (CN)
(74) Representative: dompatent
(86) International application number: PCT/CN2024/126934
(87) International publication number: WO 2025/087311

(57) **Abstract**

The present disclosure provides a pharmaceutical composition of a sulfonylurea derivative. Specifically, the present disclosure provides a pharmaceutical composition comprising a compound represented by formula (I) or a pharmaceutically acceptable salt thereof, a stabiliser, and a buffering agent. The composition has excellent stability. =

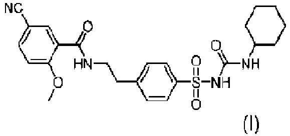

## Description

The present application claims the priority of Chinese patent application 2023113828300 filed on October 24, 2023. This Chinese patent application is incorporated herein by reference in its entirety.

### Technical Field

The present disclosure belongs to the field of pharmaceutical formulations, and in particular relates to a pharmaceutical composition of a sulfonylurea derivative and a preparation method therefor.

### Background Art

Cerebral stroke, also known as "apoplexy" or "cerebralvascular accident (CVA)", is an acute cerebrovascular disease and refers to a group of diseases that results in brain tissue damage from the sudden rupture of cerebral blood vessels or the failure of cerebral blood perfusion due to vascular occlusion, including ischemic and haemorrhagic strokes. The incidence of ischemic stroke is higher than that of haemorrhagic stroke, accounting for 60% to 70% of all cerebral strokes. Ischemic stroke can be caused by occlusion and stenosis of the internal carotid arteries and vertebral arteries, which mostly occurs in individuals over 40 years of age, and is more common in males than females, and may be fatal in severe cases. The mortality from haemorrhagic stroke is higher. Surveys show that cerebral stroke has become the leading cause of death in both urban and rural areas of China and the primary cause of disability among Chinese adults. Cerebral stroke is characterized by a high incidence, a high mortality, and a high disability rate.

The most common cause of cerebral stroke is the detachment of small emboli from the inner walls of the blood vessels supplying the brain, leading to arterial occlusion, which is referred to as ischemic stroke. It may also be caused by hemorrhage from cerebral blood vessels or thrombi, which is referred to as haemorrhagic stroke. Patients with coronary heart disease complicated by atrial fibrillation are prone to developing mural thrombi on their heart valves. The detachment of the emboli can occlude the cerebral blood vessels and thus lead to ischemic stroke. Other factors include hypertension, diabetes, hyperlipidaemia, etc.

In 2018, the biopharmaceutical company Biogen conducted a Phase III clinical trial of BIIB093 (intravenous glibenclamide) for the prevention and treatment of severe cerebral oedema in patients with large hemispheric infarction (LHI). BIIB093 is a high-affinity inhibitor of the SUR1-TRPM4 (sulfonylurea receptor 1-transient receptor potential melastatin 4) channel, which is upregulated after ischemia and trauma. The opening of these channels can lead to cerebral oedema, midline shift, increased intracranial pressure, and brain herniation, resulting in permanent disability or death. BIIB093 is an experimental drug currently being developed for the prevention and treatment of severe cerebral oedema caused by LHI. CN 101932308 B discloses a lyophilised formulation comprising glibenclamide, which may contain a small amount of buffering agent (< 5 mM) or no buffering agent, and the prepared formulation has a suitable pH for injection.

WO 2022012666 relates to a series of novel sulfonylurea derivatives, wherein the compound represented by formula (I) exhibits good activity, and has a structure as shown below:

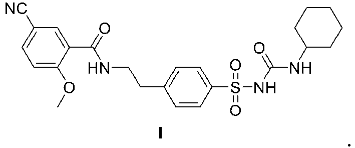

### Summary of the Invention

An objective of the present disclosure is to provide a pharmaceutical composition comprising a compound represented by formula (I) or a pharmaceutically acceptable salt thereof as an active ingredient, which composition has good formulation stability.

The present disclosure provides a pharmaceutical composition comprising a compound represented by formula (I) or a pharmaceutically acceptable salt thereof as an active ingredient, a stabiliser, and a phosphate buffering agent,

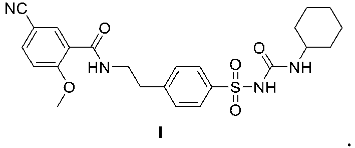

In some embodiments, examples of the phosphate buffering agent include, but are not limited to, disodium hydrogen phosphate-sodium dihydrogen phosphate, disodium hydrogen phosphate-potassium dihydrogen phosphate, sodium dihydrogen phosphate-sodium hydroxide, sodium phosphate-phosphoric acid, disodium hydrogen phosphate-citric acid, etc.

In some embodiments, the stabiliser is selected from one or more of mannitol, sucrose, trehalose, maltose, dextrose, and lactose. In some embodiments, the stabiliser is mannitol and trehalose.

In some embodiments, the concentration of the buffering agent in the pharmaceutical composition is 5-200 mM, including, but not limited to 5 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM, 105 mM, 110 mM, 115 mM, 120 mM, 125 mM, 130 mM, 135 mM, 140 mM, 145 mM, 150 mM, 155 mM, 160 mM, 165 mM, 170 mM, 175 mM, 180 mM, 185 mM, 190 mM, 195 mM, 200 mM, or any value between any two values, such as 5-100 mM, or 10-50 mM. The ratio of the compound represented by formula (I) or a pharmaceutically acceptable salt thereof as the active ingredient to the buffering agent is appropriate, which is conducive to the pH stability of the composition.

In some embodiments, the concentration of the stabiliser is 1 mg/mL to 150 mg/mL, and non-limiting examples include 1 mg/mL, 5 mg/mL, 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 70 mg/mL, 80 mg/mL, 90 mg/mL, 100 mg/mL, 110 mg/mL, 120 mg/mL, 130 mg/mL, 140 mg/mL, 150 mg/mL, and any range between these point values. In some embodiments, the concentration of the stabiliser is 10 mg/mL to 80 mg/mL.

In some embodiments, the pharmaceutical composition has a pH of 7-12, and non-limiting examples include 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9,9.0, 9.1, 9.2, 9.3,9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, 12.0, or any value between any two values. In some embodiments, the pharmaceutical composition has a pH of 8-12.

In some embodiments, the pharmaceutical composition may further comprise a pH regulator, which can be sodium hydroxide, hydrochloric acid, etc., such as sodium hydroxide. The pH regulator can be used to assist in adjusting the pH of the pharmaceutical composition to a desired range after the addition of a buffering agent.

In some embodiments, the concentration of the compound represented by formula (I) or a pharmaceutically acceptable salt thereof in the pharmaceutical composition is 0.01 mg/mL to 50 mg/mL, or 0.1 mg/mL to 20 mg/mL, or 0.3 mg/mL to 10 mg/mL, and non-limiting examples include 0.02 mg/mL, 0.05 mg/mL, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1.0 mg/mL,1.1 mg/mL, 1.2 mg/mL, 1.3 mg/mL, 1.4 mg/mL, 1.5 mg/mL, 1.6 mg/mL, 1.7 mg/mL, 1.8 mg/mL, 1.9 mg/mL, 2.0 mg/mL, 2.2 mg/mL, 2.4 mg/mL, 2.6 mg/mL, 2.8 mg/mL, 3.0 mg/mL, 4.0 mg/mL, 5.0 mg/mL, 6.0 mg/mL, 7.0 mg/mL, 8.0 mg/mL, 9.0 mg/mL, 10.0 mg/mL, 20 mg/mL, 25 mg/mL, 30 mg/mL, 35 mg/mL, 40 mg/mL, 45 mg/mL, 50 mg/mL, or any value between any two values.

The present disclosure also provides a pharmaceutical composition comprising:
0.1 mg/mL to 20 mg/mL of the compound represented by formula (I) or a pharmaceutically acceptable salt thereof;
10 mg/mL to 80 mg/mL of mannitol or trehalose as a stabiliser;
10-50 mM of a phosphate buffering agent;
and optionally a pH regulator,
wherein the composition has a pH of 8-12.

The pharmaceutical composition of the present disclosure may further comprise other pharmaceutically acceptable excipients, such as a solubilising agent, a tonicity adjuster, an adsorbent, and a complexing agent.

The pharmaceutical composition provided by the present disclosure may be a solid or a liquid, wherein a pharmaceutical composition in solid form is typically obtained by lyophilising a composition in liquid form, or a composition in liquid form can be obtained upon reconstitution. The pharmaceutical composition in liquid form may be in solution form or suspension form, preferably in solution form. In some embodiments, the liquid composition is obtained by reconstituting a lyophilised composition, or is the liquid composition prior to lyophilisation. In some embodiments, the solid composition, upon reconstitution, results in the aforementioned pharmaceutical composition.

In some embodiments, the pharmaceutical composition of any of the foregoing is a liquid formulation. The liquid formulation or reconstituted formulation of the present disclosure has good stability.

In some embodiments, after the pharmaceutical composition of the present disclosure is stored at 25°C/60% RH for 30 days, the content of the compound represented by formula (I) or a pharmaceutically acceptable salt thereof is ≥ 96%, such as ≥ 97% or ≥ 98%.

The present disclosure also provides a lyophilised formulation, wherein the formulation, upon reconstitution, forms the aforementioned pharmaceutical composition.

The present disclosure also provides a lyophilised formulation, wherein the lyophilised formulation is obtained by lyophilising the aforementioned pharmaceutical composition.

The present disclosure also provides a reconstituted solution, wherein the reconstituted solution is prepared by reconstituting the aforementioned lyophilised formulation.

The present disclosure further provides a method for preparing the reconstituted solution described above, wherein the method comprises the step of reconstituting the aforementioned lyophilised formulation, wherein the solution used for the reconstitution is selected from, but not limited to, water for injection, physiological saline or glucose solution.

The present disclosure further provides the use of the pharmaceutical composition of the present disclosure in the preparation of a medicament for treating diseases and conditions affected by neuronal injury.

The present disclosure further provides the use of the pharmaceutical composition of the present disclosure in the preparation of a medicament for treating acute cerebral stroke, traumatic brain injury, spinal cord injury, myocardial infarction, shock, organ ischemia, ventricular arrhythmias, ischemic injury, hypoxia/ischemia, and Parkinson's disease.

The present disclosure provides a method for preparing the aforementioned pharmaceutical composition, comprising the step of mixing the compound represented by formula (I) or a pharmaceutically acceptable salt thereof with a stabiliser and a buffering agent.

In some embodiments, the method further comprises the steps of adjusting the pH and performing lyophilisation.

The pharmaceutical composition provided by the present disclosure has excellent stability. The lyophilised formulation has a minimal pH shift upon reconstitution and can be injected directly after dilution. The buffering agent-free pharmaceutical composition has poor stability, and the lyophilised formulation exhibits a significant decrease in pH upon reconstitution, posing a risk of precipitation of the active substance.

As used herein, the term "about" or "approximately" means that the numerical value is within an acceptable error range for a particular value as determined by a person of ordinary skill in the art, which will depend in part on how the value is measured or determined (i.e., the limitations of the measurement system). For example, "about" may mean within 1 or more than 1 standard deviation, per the practice in the art. Alternatively, "about" or "substantially comprising" may mean a range of up to 20%. In addition, particularly with respect to biological systems or processes, the term may mean within at most an order of magnitude or within at most 5-fold of a value. Unless otherwise stated, when a particular value appears in the present application and claims, the meaning of "about" or "substantially comprising" shall be assumed to be within an acceptable error range for that particular value.

The numerical values in the present disclosure are measured values from instruments or calculated values derived from instrument measurements, which have a certain degree of error. In general, plus or minus 10% is within a reasonable error range. Of course, the context in which the numerical value is used needs to be considered. For example, with regard to the content of total impurities, the change in the error of the numerical value after measurement does not exceed plus or minus 10%, and may be plus or minus 9%, plus or minus 8%, plus or minus 7%, plus or minus 6%, plus or minus 5%, plus or minus 4%, plus or minus 3%, plus or minus 2% or plus or minus 1%, preferably plus or minus 5%.

"Weight-to-volume ratio (w/v)" as used herein refers to the weight (in g) of the component contained per 100 mL of a liquid system, i.e., g/100 mL.

### Detailed Description of Embodiments

The present disclosure is further illustrated in detail by the following examples. These examples are merely for illustrative purposes and are not intended to limit the scope of the present disclosure.

### Example 1

Experimental method: A sodium hydroxide solution or phosphate buffer at the prescribed pH was prepared, 80% of the prescribed amount of the aforementioned solution was taken, and the compound represented by formula (I) was weighed and added to the solution. The mixture was stirred until completely dissolved. A prescribed amount of mannitol or trehalose was then added, and the mixture was stirred until completely dissolved. The sodium hydroxide solution or phosphate buffer at the prescribed pH was added to make up the total amount. The mixture was mixed uniformly, sterilised by filtration, and filled into vials, which were then partially stoppered, lyophilised, and capped.

**Table 1 Components of each composition**

| | Formulation 1 | Formulation 2 | Formulation 3 |
|---|---|---|---|
| Compound represented by formula (I) | 3 mg | 3 mg | 3 mg |
| Mannitol (w/v) | 3% | 3% | |
| Trehalose (w/v) | | | 3% |
| 20 mM phosphate solution at pH 11.5 | / | Q.S. | Q.S. |
| Sodium hydroxide solution at pH 11.5 | Q.S. | / | / |
| Water for injection | To 3 ml | To 3 ml | To 3 ml |
| pH before lyophilisation | 10.94 | 10.99 | 10.95 |

### Chemical stability test of lyophilised formulations:

The prepared samples were placed at 40°C for 30 days, and the investigation results are shown in Table 2. The results showed that Formulation 1, which contained no buffering agent, exhibited a rapid decrease in the purity of the active ingredient and poor stability, and that Formulations 2 and 3, which contained buffering agents, showed favourable stability.

**Table 2**

| Placement condition | Placement time (day) | Formulation 1 Active ingredient purity (%) | Formulation 2 Active ingredient purity (%) | Formulation 3 Active ingredient purity (%) |
|---|---|---|---|---|
| | 0 | 98.52 | 98.47 | 98.56 |
| 40°C/75% RH | 7 | 98.52 | 98.47 | 98.56 |
| | 14 | 98.3 | 98.26 | 98.56 |
| | 30 | 98.08 | 98.34 | 98.45 |

### pH stability test of reconstituted solutions:

The samples were reconstituted with water for injection, and the investigation results are shown in Table 3. The results showed that Formulation 1, which contained no buffering agent, exhibited significant pH fluctuation upon reconstitution, while Formulations 2 and 3 had a relatively stable pH.

**Table 3**

| **Formulation no.** | **pH of reconstituted solution** | **40°C** | | |
|---|---|---|---|---|
| | | **7 d** | **14 d** | **30 d** |
| Formulation 1 | 9.55 | 9.71 | 9.93 | 9.90 |
| Formulation 2 | 10.16 | 10.31 | 10.32 | 10.29 |
| Formulation 3 | 10.36 | 10.4 | 10.39 | 10.40 |

### Example 2

According to the method of Example 1, pre-lyophilisation solutions at different pH, and lyophilised formulations were prepared.

**Table 4 Components of each composition**

| | Formulation 4 | Formulation 5 | Formulation 6 |
|---|---|---|---|
| Compound represented by formula (I) | 3 mg | 3 mg | 3 mg |
| Mannitol (w/v) | 3% | 3% | 3% |
| 20 mM Phosphate solution | Q.S. | Q.S. | Q.S. |
| Water for injection | To 3 ml | To 3 ml | To 3 ml |
| pH before lyophilisation | 10.91 | 10.32 | 9.44 |
| pH of reconstituted solution (10 µg/ml) | 7.65 | 7.42 | 7.35 |
| pH of reconstituted solution (5 µg/ml) | 7.17 | 7.11 | 7.02 |

### Chemical stability test of lyophilised formulations:

The prepared samples were placed at 40°C for 30 days, and the investigation results are shown in Table 5. The results showed that all the samples exhibited good stability.

**Table 5**

| Placement condition | Placement time | Formulation 4 | Formulation 5 | Formulation 6 |
|---|---|---|---|---|
| N/A | Initial | 97.62 | 97.77 | 97.75 |
| 40°C | 7 days | 97.51 | 97.56 | 97.60 |
| | 15 days | 97.44 | 97.61 | 97.69 |
| | 1 M | 97.61 | 97.78 | 97.77 |

### pH stability test of reconstituted solutions:

Samples prepared from the lyophilisation solutions at different pH were placed at 40°C for 30 days, and the pH of the reconstituted solutions was investigated. The investigation results are shown in Table 6. The results showed that the pH of all samples was relatively stable.

**Table 6**

| **Formulation batch no.** | pH of reconstituted solution after lyophilisation | 40°C | | |
|---|---|---|---|---|
| | | 7 d | 14 d | 30 d |
| Formulation 4 | 9.78 | N/A | 9.92 | 9.74 |
| Formulation 5 | 9.10 | 9.19 | 9.52 | 9.03 |
| Formulation 6 | 8.64 | 8.64 | 8.72 | 8.56 |

**Experimental conclusions:** All the compositions exhibited good stability in all tests. In addition, the pH of the solutions of all compositions was nearly neutral after reconstitution and dilution with a small amount of water, making them suitable for clinical use.

### Example 3

According to the method of Example 1, pre-lyophilisation solutions with different mannitol contents, and lyophilised formulations were prepared.

**Table 7 Components of each composition**

| | Formulation 7 | Formulation 8 |
|---|---|---|
| Compound represented by formula (I) | 3 mg | 3 mg |
| Mannitol (w/v) | 2% | 4% |
| 20 mM Phosphate solution | Q.S. | Q.S. |
| Water for injection | To 3 ml | To 3 ml |

### Chemical stability test of lyophilised formulations:

The prepared samples were placed at 40°C for 30 days, and the investigation results are shown in Table 8. The results showed that all the samples exhibited good stability.

**Table 8**

| **Placement condition** | **Placement time** | **Formulation 7** | **Formulation 8** |
|---|---|---|---|
| 0 day | After lyophilisation | 98.10 | 97.66 |
| | 8 days | 98.07 | 97.49 |
| 40°C | 15 days | 98.02 | 97.81 |
| | 1 M | 97.99 | 97.57 |

### pH stability test of reconstituted solutions:

The samples were reconstituted with water for injection, and the pH of the reconstituted solution was measured. The investigation results are shown in Table 9. The results showed that the pH of all samples after reconstitution was relatively stable.

**Table 9**

| **Formulation no.** | **Lyophilised solution** | **40°C** | | |
|---|---|---|---|---|
| | | **8 d** | **15 d** | **30 d** |
| Formulation 7 | 8.83 | 8.80 | 8.39 | 8.69 |
| Formulation 8 | 8.81 | 8.82 | 8.62 | 8.73 |

### Experimental conclusions: All the compositions exhibited good stability in all tests.

### Example 4

According to the method of Example 1, pre-lyophilisation solutions with different buffering agent concentrations, and lyophilised formulations were prepared.

**Table 10 Components of each composition**

| | Formulation 9 | Formulation 10 |
|---|---|---|
| Compound represented by formula (I) | 3 mg | 3 mg |
| Mannitol (w/v) | 3% | 3% |
| Phosphate solution | 10 mM | 30 mM |
| Water for injection | To 3 ml | To 3 ml |
| pH before lyophilisation | 10.32 | 10.44 |

### Chemical stability test of lyophilised formulations:

The prepared samples were placed at 40°C for 30 days, and the investigation results are shown in Table 11. The results showed that all the samples exhibited good stability.

**Table 11**

| Placement time | | Formulation 9 | Formulation 10 |
|---|---|---|---|
| After lyophilisation | | 97.74 | 97.45 |
| 40°C | 7 days | 97.82 | 97.24 |
| | 17 days | 97.64 | 97.2 |
| | 30 days | 97.63 | 97.78 |

### Example 5

According to the method of Example 1, pre-lyophilisation solutions with different types of buffering agents, and lyophilised formulations were prepared.

**Table 12 Components of each composition**

| | Formulation 11 | Comparative formulation 1 | Comparative formulation 2 | Comparative formulation 3 |
|---|---|---|---|---|
| Compound represented by formula (I) | 3 mg | 3 mg | 3 mg | 3 mg |
| Mannitol (w/v) | 3% | 3% | 3% | 3% |
| Buffer system | 20 mM NaH₂PO₄-NaOH | 20 mM Lysine hydrochloride-NaOH | 20 mM Glycine-NaOH | 20 mM Arginine-HCl |
| Water for injection | To 3 ml | To 3 ml | To 3 ml | To 3 ml |
| pH before lyophilisation | 10.5 | 10.5 | 10.5 | 10.5 |

### 1) Results of pH buffering capacity investigation

| | Formulation 11 | Comparative formulation 1 | Comparative formulation 2 | Comparative formulation 3 |
|---|---|---|---|---|
| Excipient stock solution | 7.0 | 7.0 | 6.9 | 7.1 |
| API stock solution | 12.2 | 12.2 | 12.2 | 12.2 |
| After mixing of active ingredient and excipient | 10.8 | 9.2 | 9.7 | 9.2 |

Under the condition of comparable concentrations and pH of the active ingredient and excipient, the mixture of the active ingredient and excipient showed that, except for the phosphate buffer group (Formulation 11), all other groups exhibited a significant decrease in pH, indicating that the phosphate buffer at the same molar concentration has superior buffering capacity compared with that of the other groups.

### 2) Impurity content (%) of lyophilised formulations under 25°C accelerated stability conditions

| Time | Formulation 11 | Comparative formulation 1 | Comparative formulation 2 | Comparative formulation 3 |
|---|---|---|---|---|
| 0 day | 0.50 | 0.86 | 1.29 | 0.66 |
| 15 days | 0.55 | 4.91 | 4.23 | 1.89 |

It can be seen that the impurity variation of the phosphate buffer group (Formulation 11) was significantly lower than that of all other groups, indicating good stability.

## Claims

1. A pharmaceutical composition, **characterized by** comprising a compound represented by formula (I) or a pharmaceutically acceptable salt thereof as an active ingredient, a stabiliser, and a phosphate buffering agent,

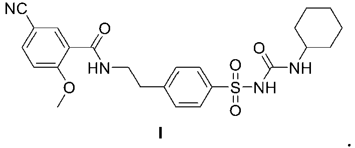

2. The pharmaceutical composition according to claim 1, **characterized in that** the stabiliser is selected from one or more of mannitol, sucrose, trehalose, maltose, dextrose, and lactose, preferably mannitol or trehalose.

3. The pharmaceutical composition according to claim 1 or 2, **characterized in that** the pharmaceutical composition further comprises a pH regulator, preferably hydrochloric acid or sodium hydroxide.

4. The pharmaceutical composition according to any one of claims 1-3, **characterized in that** the concentration of the buffering agent in the pharmaceutical composition is 5-200 mM, preferably 5-100 mM, and more preferably 10-50 mM.

5. The pharmaceutical composition according to any one of claims 1-4, **characterized in that** the concentration of the stabiliser is 1 mg/mL to 150 mg/mL, preferably 10 mg/mL to 80 mg/mL.

6. The pharmaceutical composition according to any one of claims 1-5, **characterized in that** the composition has a pH of 7-12, preferably 8-12.

7. The pharmaceutical composition according to any one of claims 1-6, **characterized in that** the concentration of the compound represented by formula (I) or the pharmaceutically acceptable salt thereof is 0.01 mg/mL to 50 mg/mL, preferably 0.1 mg/mL to 20 mg/mL, and more preferably 0.3 mg/mL to 10 mg/mL.

8. A pharmaceutical composition, **characterized by** comprising:
0.1 mg/mL to 20 mg/mL of the compound represented by formula (I) or a pharmaceutically acceptable salt thereof;
10 mg/mL to 80 mg/mL of mannitol or trehalose as a stabiliser;
10-50 mM of a phosphate buffering agent;
and optionally a pH regulator,
wherein the composition has a pH of 8-12.

9. A lyophilised formulation, **characterized in that** the lyophilised formulation, upon reconstitution, forms the pharmaceutical composition according to any one of claims 1-8.

10. A lyophilised formulation, **characterized in that** the lyophilised formulation is obtained by lyophilising the pharmaceutical composition according to any one of claims 1-8.

11. A reconstituted solution, **characterized in that** the reconstituted solution is prepared by reconstituting the lyophilised formulation according to claim 9 or 10.

12. An article of manufacture, **characterized by** comprising a container containing the pharmaceutical composition according to any one of claims 1-8, the lyophilised formulation according to claim 9 or 10, or the reconstituted solution according to claim 11.

13. Use of the pharmaceutical composition according to any one of claims 1-8, the lyophilised formulation according to claim 9 or 10, the reconstituted solution according to claim 11, or the article of manufacture according to claim 12 in the preparation of a medicament for treating diseases and conditions affected by neuronal injury.

14. Use of the pharmaceutical composition according to any one of claims 1-8, the lyophilised formulation according to claim 9 or 10, the reconstituted solution according to claim 11, or the article of manufacture according to claim 12 in the preparation of a medicament for treating acute cerebral stroke, traumatic brain injury, spinal cord injury, myocardial infarction, shock, organ ischemia, ventricular arrhythmias, ischemic injury, hypoxia/ischemia, and Parkinson's disease.
